# EUROPEAN PATENT APPLICATION

(11) **EP 3 913 042 A1**
(43) Date of publication of application: **24.11.2021**
(21) Application number: 20175244.1
(22) Date of filing: 18.05.2020
(51) Int. Cl.: C12M 1/32, C12M 1/00, C12M 1/34

(54) **ANALYSIS DEVICE**

(71) Applicant: Incyton GmbH, 82152 Planegg (DE)
(72) Inventor: Herz, Helmut, 80997 München (DE); Strehle, Martin, 81476 München (DE); Wolf, Peter, 81243 München (DE); Moll, Matthias, 81379 München (DE)
(74) Representative: Weiss, Wolfgang

(57) **Abstract**

The present invention relates to a device and a method for the analysis of cultured cells, comprising a plurality of culture vessels, optical sensors and a detection system with detectors for determining a physico-chemical parameter in a culture vessel and for imaging the morphology of cells in a vessel. The device comprises a microscope adapted to be moved and having an optical path provided with switching means to switch between an optical path for determining a physico-chemical parameter and an optical path for imaging the morphology of cells.

## Description

The present invention relates to a device and a method for the analysis of cultured cells.

### Background

In pharmacological and medical research, assays using living cells have become highly important. For this purpose, numerous automated devices for the analysis of cultured cells were developed in the last decades. For example, such devices are described in EP-A-0881490, EP-A-0938383 and EP-0760095.

The culture of living cells a complex procedure and susceptible to disturbances from external sources. Thus, it is an object of the present invention to provide devices and methods for analyzing cultured cells, which allow measuring of multiple cellular parameters under conditions wherein the cells are protected from disturbances.

### Summary of the Invention

According to the present invention, a device and a method for the analysis of cultured cells are provided. The device novel device can be used as a multi-sensor cell analysis platform. It comprises a plurality of independent cultivation vessels, e.g. in wells of a sensor-equipped micro-titer plate, and allows monitoring and analyzing different parameters simultaneously in real-time and label-free. The cells are cultured under controlled climatic conditions and remain at a specific location within the device throughout a measurement cycle.

The device of the present invention allows a constant monitoring of cellular metabolism by measuring physico-chemical parameters such as pH and/or the oxygen content. In parallel, cellular characteristics are determined by electrical and optical measurements, e.g. by measuring cellular impedance and imaging of the cellular morphology. These measurements are performed automatically in a fully controllable environment. Investigation protocols may be performed in parallel in a plurality of cultivation vessels comprising test cells and/or references. Culture media and test substances are supplied and removed by automated supply and/or removal means, e.g. by an automated pipetting robot. This life support fluidic system allows cells to be monitored and treated in the sensor plate for days up to several weeks in vital conditions and free of disturbance and contamination. Thus, the device provides long-term real-time measurements. Sequences of experiments may be programmed and raw and processed data generated.

A first aspect of the present invention relates to a device for the analysis of cultured cells comprising:
a) a plurality of cultivation vessels adapted for culturing cells, particularly for culturing adherent cells,
b) a climatic chamber in which the cultivation vessels (a) are positioned,
c) a supply and/or removal system for supplying and/or removing materials, e.g. fluids, including cultivation media and/or test compounds to or from the cultivation vessels (a), and optionally at least one reservoir for supply materials and for waste,
d) a light source for transmitting radiation to the cultivation vessels (a),
e) at least one optical sensor which is responsive to a physico-chemical parameter in a cultivation vessel (a), wherein the physico-chemical parameter is particularly selected from physico-chemical parameters indicative for the metabolic condition of cells, such as pH, oxygen content, and optionally at least one further parameter,
f) at least one electrode for measuring the impedance of cells in a cultivation vessel (a),
g) a detector or detector system for receiving radiation from a cultivation vessel (a), wherein the detector or detector system is adapted for (i) determining a physico-chemical parameter in a cultivation vessel (a) and (ii) imaging the morphology of cells in a cultivation vessel (a), and
h) a microscope for providing an optical path between a cultivation vessel (a) and the detector or detector system (g), wherein the microscope is adapted to be moved between a plurality of positions within the device, wherein an individual microscope position is attributed to an individual cultivation vessel (a), and wherein the microscope comprises switching means for providing a switch between an optical path for determining a physico-chemical parameter and an optical path for imaging the morphology of cells.

A further aspect of the present invention relates to a method of cultivating and analyzing cells, comprising the steps:
a) cultivating cells, particularly adherent cells, in a plurality of cultivation vessels, wherein said cultivation vessel are positioned in a climatic chamber,
b) supplying and/or removing materials, e.g. fluids, including cultivation media and/or test compounds to or from the cultivation vessels,
c) transmitting radiation from a light source to the cultivation vessels,
d) optically determining at least one physico-chemical parameter in the cultivation vessels using an optical sensor which is responsive to said physico-chemical parameter, wherein the physico-chemical parameter is particularly selected from physico-chemical parameters indicative for the metabolic condition of cells, such as pH, oxygen content, and optionally at least one further parameter,
e) optically imaging the morphology of cells in the cultivation vessels, and
f) measuring the impedance of cells in the cultivation vessels,
wherein radiation from a cultivation vessel (a) is transmitted to a detector or detector system, which is adapted for (i) determining a physico-chemical parameter in a cultivation vessel (a) and (ii) imaging the morphology of cells in a cultivation vessel (a),
wherein an optical path is provided between a cultivation vessel and the detector or detector system,
wherein the optical path runs via a microscope, which is moved between a plurality of positions within the device, wherein an individual microscope position is attributed to an individual cultivation vessel (a), and
wherein the microscope comprises switching means for providing a switch between an optical path for determining a physico-chemical parameter and an optical path for imaging the morphology of cells.

### Detailed description

The device of the present invention comprises a plurality of cultivation vessels (a) adapted for culturing cells, e.g. at least 2 and up to about 100 or more cultivation vessels. In certain embodiments, the device comprises at least about 6 and up to about 100 cultivation vessels, particularly at least up to about 10 and up to about 30 cultivation vessels and more particularly 24 cultivation vessels. The cells are cultured in the cultivation vessels. Typically, the culture volume, i.e. the combined volume of cells and culture medium, is about 0.1 ml to about 1.5 ml, particularly about 0.5 ml to about 1 ml and more particularly about 0.7 to about 0.9 ml.

In certain embodiments, the cultivation vessels are located in a plane oriented in x-y direction within the device. In certain embodiments, the cultivation vessels are wells in at least one contiguous plate, particularly in at least one contiguous micro-titer plate. Micro-titer plates containing wells suitable for cell-culture are e.g. available from InnoME Munich, München, Germany, and Erwin Quarder Systemtechnik, Espelkamp, Germany, for example, the product v14-komplettsystem.

In certain embodiments, the cell cultivation vessels comprise several chambers, e.g. an inlet chamber for introduction of materials, e.g. fresh media or test compounds, a reaction chamber for cultivating the cells, and an outlet chamber for the removal of waste materials.

The cultured cells may be any cells, which can be cultured in vitro, e.g. cell lines, primary cells or cell tissues. The cells may be cultured as such or in the presence of matrix, e.g. a three-dimensional matrix for supporting the cells. For example, the cells may be eukaryotic cells such animal cells, particularly mammalian cells including human cells, e.g. adherent cell lines such as HEK293 (ATCC CRL-1573), NIH/3T3 (ATCC CRL-1658), L-929 (ATCC CCL-1), HepG2 (ATCC HB-8065), or Vero E6 (ATCC CRL-1586), plant cells or fungal cells, or prokaryotic cells such as bacterial cells. In particular embodiments, the cells are adherent cells. It should be noted, however, that the device is also suitable for culturing suspended non-adherent cells.

In particular embodiments, the cultivation vessels or a contiguous plate containing cultivation vessels are located at a stationary position within the device. Thus, the location of the cultivation vessels and the cells within the cultivation vessels can remain constant within the device throughout a measurement cycle. Thereby, disturbance caused by moving cultivation vessels within the device are avoided.

Further, the device comprises a climatic chamber (b) which encompasses the cultivation vessels (a). The climatic chamber defines the external environment for the cells. It may provide a closed environment, which is sealed from the environment outside of the device and optionally from interior parts of the device, thereby limiting and/or prohibiting access of gaseous and/or fluidic contaminants to the cells. The climatic chamber may comprise a separation element, e.g. a slide element, for physically separating the culture vessels from other interior parts of the device, e.g. from the supply and/or removal system (c) and/or from the microscope (h), wherein the separation may be temporal or permanent throughout a measurement cycle. The separation element may be actuated manually or automatically, by means of a motor.

The climatic chamber (b) is provided with a control element adapted for measuring, maintaining and/or adjusting environmental parameters in the climatic chamber such as (i) temperature, (ii) gas atmosphere, e.g. the partial pressure of oxygen and/or the partial pressures of components of a hypoxia gas mixture comprising nitrogen and/or noble gases, and/or (iii) humidity. More particularly, the climatic chamber may comprise sensors adapted for measuring relevant environmental parameters, an external reservoir for providing atmospheric constituents, e.g. gas and/or humidity to the climatic chamber, and a temperature control element. Further, the climatic chamber may comprise a ventilation filter element and/or a pressure control adapted for pressure adjustment and/or maintenance, particularly for adjusting and/or maintaining super-atmospheric, atmospheric or sub-atmospheric pressure as required.

The device of the present invention comprises a supply and/or removal system (c) for supplying and/or removing materials to or from the cultivation vessels (a). The supply and/or removal system may be a pipetting system, particularly an automated pipetting robot comprising a multi-pipette head. In certain embodiments, the pipette head comprises a number of pipettes corresponding to the number of cultivation vessels (a). Further, the individual pipettes in a multi-pipette head may be spatially arranged such that parallel pipetting to and from a plurality of cultivation vessels, e.g. to and from all cultivation vessels is possible. Additionally, the supply and/or removal system may comprise at least one reservoir for supply materials, e.g. fluids, including cultivation media and/or test compounds and/or for waste materials. The reservoir may include a plurality of vessels, e.g. as wells of a micro-titer-plate. Suitable reservoir plates are e.g. available from Ritter GmbH, Schwabmünchen, Germany.

The device is adapted for performing optical measurements and comprises at least one light source (d) for transmitting radiation to the cultivation vessels (a). The light source may be adapted for providing radiation in the IR, VIS or UV range as desired. In certain embodiments, the light source may be a laser or LED light source capable of emitting light at one or more desired wavelengths.

Further, the device comprises at least one optical sensor (e) which is responsive to a physico-chemical parameter in a cultivation vessel (a). Typically, different types of optical sensors are used for the measurement of different parameters. For example, a first type of optical sensor is used for the measurement of a first parameter, and a second type of optical sensor is used for the measurement of a second parameter. In certain embodiments, an individual cultivation vessel, e.g. each cultivation vessel is provided with its own optical sensor(s). The physico-chemical parameters are particularly selected from parameters indicative for the metabolic condition of cells, such as pH of the culture medium, oxygen content of the culture medium, and optionally at least one further parameter such as CO₂. Monitoring the pH of the culture medium over at least a part of the measurement cycle may provide the extra-cellular acidification rate (ECAR) which is a relevant parameter of cell viability. Monitoring the oxygen content of the cultivation medium over at least a part of the measurement cycle may provide the oxygen consumption rate (OCR) which is a further relevant parameter of cell viability. Methods of measuring ECAR and OCR are e.g. described in US2010/0124761A, which is herein incorporated by reference.

Optical sensors for measuring parameters of cultured cells are well known in the art. They can be used for various optical measurement methods such as reflection, absorption, evanescent wave, luminescence, e.g. fluorescence and/or phosphorescence, chemiluminescence or surface plasmon resonance. According to certain embodiments, the optical sensor is a sensor for the measurement of luminescence, e.g. fluorescence. Typically, an optical sensor includes a sensor compound, which is responsive for a parameter to be measured. The sensor compound is placed in contact with the culture medium in the cultivation vessel. In certain embodiments, the sensor compound is immobilized, e.g. within a polymer matrix in the cultivation vessel. Radiation from a light source is transmitted via an optical path into the cultivation vessel e.g. by means of an optical fiber, for excitation of the sensor compound. Emitted radiation from the sensor compound, e.g. radiation generated by chemiluminescence, transmitted via an optical path from the cultivation vessel to an optical detector or detector system. In certain embodiments, sinus amplitude-modulated radiation for fluorescence stimulation is passed by means of an optical fiber to the sensor compound in the cultivation vessel and the fluorescence signal generated by the sensor compound is passed to an optical detector or detector system. Suitable optical sensors are e.g. the pH mini or oxygen mini sensors available from Presens GmbH, Regensburg, Germany.

The device of the present invention further comprises at least one electrode (f) for measuring the impedance of cells in a cultivation vessel. In certain embodiments, an individual cultivation vessel, e.g. each cultivation vessel is provided with its own electrode(s). Typically, an electrode is in contact with the culture medium and the cells present therein. For example, a cultivation vessel may comprise at least two electrodes located at its bottom or walls, wherein the electrodes are connected with an electrical detector via conductive tracks. Other types of electrodes are e.g. described in WO 2004/010102 and WO 2004/010103, which are herein incorporated by reference.

The device of the present invention also comprises an optical detector or detector system (g) for receiving radiation from a cultivation vessel (a). The optical detector or detector system is adapted for determining optical signals of two different types. On the one hand, the optical detector or detector system is adapted for measuring a physico-chemical parameter by means of the optical sensor (e) as described above. On the other hand, the optical detector or detector system is adapted for imaging the morphology of the cultured cells, e.g. by means of a microscope (h) as described below. Typically, the optical detector or detector system is adapted for quantitatively determining radiation emitted from the sensor compound of the optical sensor (e). Further, the detector or detector system comprises means for recording an image from a microscope (h). In certain embodiments, the optical detector or detector system comprises a CCD camera or a photon avalanche diode.

The device also comprises a microscope (h) for imaging of the cell morphology. Images of cells in a cultivation vessel are transmitted via an optical path to the optical detector or detector system (g). In certain embodiments, the device comprises a single microscope (h). For example, a microscope as described in DE 11 2004 000 340 T may be used, which is herein incorporated by reference.

The microscope is adapted for movement within the device, particularly for movement in x- and y-direction within a plane at least substantially parallel to a plane where the plurality of cultivation vessels is located. An individual microscope position may be attributed to an individual cultivation vessel. Thus, the microscope may be adapted for imaging a single cultivation vessel in each individual microscope position. The microscope is movable to a plurality of different positions, particularly to a number of different positions, which corresponds to the number of cultivation vessels. In such an embodiment, the microscope is adapted for providing an individual optical path between an individual cultivation vessel and the optical detector or detector system (g).

According to the present invention, an optical path between the cultivation vessel and the optical detector or detector system is provided through the microscope. The microscope comprises switching means for providing a switch between an optical path for determining a physico-chemical parameter by means of the optical sensor and an optical path for imaging the morphology of cells. The switching means allows real-time and substantially simultaneous determination of physico-chemical parameters and imaging of cell morphology.

There are several embodiments for switching optical paths within a measurement cycle. In certain embodiments, the device is adapted for performing a measurement cycle comprising the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels, particularly for all cultivation vessels. In particular embodiments, the sequence of steps (i), (ii) and (iii) is repeated at least once until the measurement cycle is performed.

In further embodiments, the device may be adapted for performing a measurement cycle comprising the steps (i) determining one or more physico-chemical parameters in a plurality of cultivation vessels, particularly in all cultivation vessels, by moving the microscope between a plurality of positions and (ii) imaging the morphology of cells in a plurality of cultivation vessel, particularly in all cultivation vessels, by moving the microscope between a plurality of positions; wherein steps (i) and (ii) may be performed in any order.

In still further embodiments, the device may be adapted for performing a measurement cycle comprising the steps (ia) determining a first physico-chemical parameter in a plurality of cultivation vessels, particularly in all cultivation vessels, by moving the microscope between a plurality of positions; (ib) determining a second physico-chemical parameter in a plurality of cultivation vessels, particularly in all cultivation vessels, by moving the microscope between a plurality of positions and (ii) imaging the morphology of cells in a plurality of cultivation vessels, particularly in all cultivation vessels, by moving the microscope between a plurality of positons; wherein steps (ia), (ib) and (ii) may be performed in any order.

The device comprises a switching means, e.g. a switchable optical window for switching between two different optical paths. A first optical path provides transmission of signals from the measurement of physico-chemical parameters to the optical detector or detector system and a second optical path provides transmission of signals from the imaging of cell morphology to the optical detector or detector system. The switching between optical paths may be effected by switching optical elements, wherein a first optical path comprises a first optical element and a second optical path comprises a second optical element. Switching from the first optical path to second optical path may comprise moving the first optical element out of the optical path and the second optical element into the optical path. Conversely, switching from the second optical path to first optical path may comprise moving the second optical element out of the optical path and the first optical element into the optical path. The first and the second optical elements may be mounted on a movable sliding element. Actuation of the sliding element will move the elements into or out from the path, as desired. For example, the first optical element comprises a prism mirror and/or the second optical element comprises an optically semi-transparent filter or mirror.

The switching means may be adapted for providing a determination of the physico-chemical parameter and the imaging of cell morphology, which is substantially simultaneous and which is performed in the same location within the cultivation vessel, thereby allowing minimal temporal and/or spatial offset. The temporal offset may be e.g. about 2 s or less, particularly between about 0.5 s and about 1 s. The spatial offset may be about 2 mm or less, particularly between about 0.1 mm and about 1 mm.

In a particular embodiment, the device of the present invention is characterized by the following features:

| **General technical data** | | |
|---|---|---|
| Dimensions (W/H/D) | mm | 670 x 1690 x 780 |
| Weight | kg | 250 |
| Electrical supply | VAC/Hz | 230 ± 10 / 50-60 |

| **Environmental conditions for storage and operation** | | |
|---|---|---|
| Temperature range | °C | 5 - 40 |
| Relative humidity, maximum | % rH | 80 |

| **Incubator** | | |
|---|---|---|
| Setting Temperature range | °C | +18 to +50 |
| Setting Temperature step | °C | 0.1 |
| Setting relative Humidity range | % rH | 30 - 80 |
| Setting O2 range | % | 1 - 21 |

| **Robot** | | |
|---|---|---|
| Pipetting volume | µl | 5 - 250 |
| Amount of channels /tips | - | 24 |

| **Microscope imaging system** | | |
|---|---|---|
| Objective magnification | x | 20 |
| Z - drive type | - | VoiceCoil |
| Focus range / resolution | µm | 6800 / 0.002 |
| Camera resolution | MPx | 12.3 |
| Pixel size | µm | 3.45 |

| **Cellular Impedance** | | |
|---|---|---|
| Throughput (wells per second) | 1 / s | 14 |
| Measuring frequency | kHz | 10 |
| Measuring range impedance Z | Ω | 10 - 5000 |
| Measuring range Resistance R | Ω | 10 - 5000 |
| Measuring range capacity C | nF | 0.3 - 3000 |
| Measuring error | % | <2 |

| **pH and pO2 single channel measuring module** | | |
|---|---|---|
| | PO₂ | pH |
| Measuring range | 0 - 50% | 6.0 - 8.5 pH |
| Resolution | ± 0.4 % pO₂ | ± 0.05 pH |
| Signal drift | 0.2 % / week | 0.1 pH / week |
| Accurateness | ± 1.0 % at 20.9 % pO₂ | ±0.2 % at pH7 |

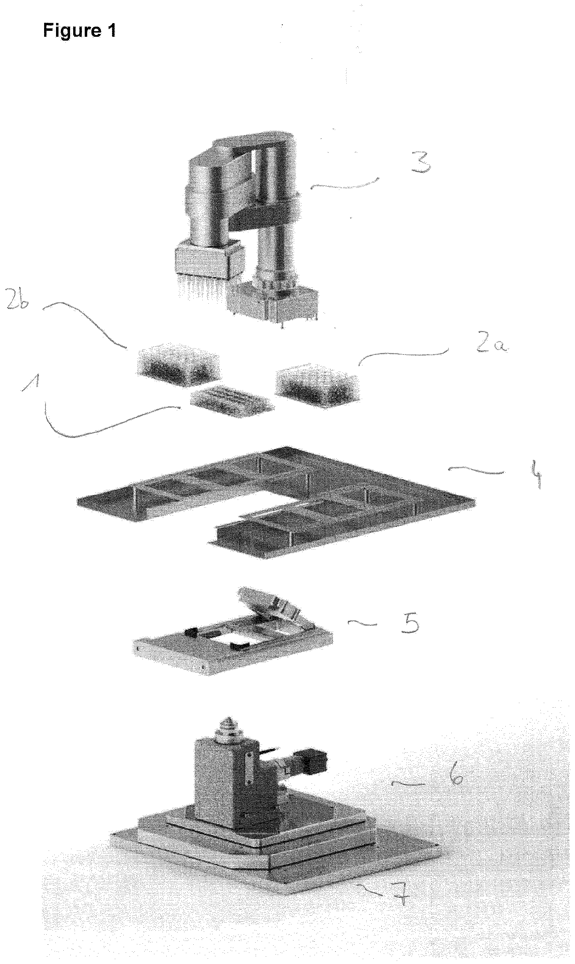

In Figure 1, specific embodiment of the device is schematically depicted. Accordingly, the device comprises a 24 well micro-titer plate (1) with integrated optical sensors. Further, reservoirs (2a, 2b) for supply fluids may be present. Supply and removal of fluids into and from cultivation vessels is performed by a 24 channel pipetting robot (3). The cultivation vessel plate and the reservoir plates may be arranged on a rack (4) including a plate slot and impedance housing (5). A microscope (6) is mounted on a stage (7) which allows movement in x- y- direction.

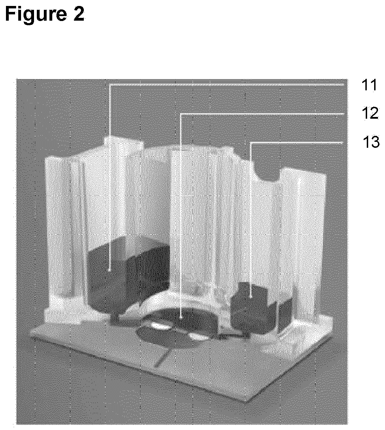

A cultivation vessel is shown in more detail in Figure 2. It comprises a media inlet (11), a micro reaction chamber (12) and a waste media outlet (13) to ensure constant nutrition for the cultivated cells. The micro reaction chamber includes with sensors, e.g. optical pH and pO₂ sensors and an electrical impedance sensor as well as a microscopy window for imaging.

Further, a method of cultivating and analyzing cells is provided which allows switching between determination of a physico-chemical parameter using an optical sensor and imaging of cell morphology using a microscope. In certain embodiments, the method involves the use of a device as described above in detail.

In certain embodiments of the method, performing a measurement cycle comprises the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels, particularly for all cultivation vessels. In particular embodiments, the sequence of steps (i), (ii) and (iii) is repeated at least once until the measurement cycle is performed.

In further embodiments, performing a measurement cycle comprises the steps (i) determining one or more physico-chemical parameters in a plurality of cultivation vessels, particularly in all cultivation vessels, by moving the microscope between a plurality of positions and (ii) imaging the morphology of cells in a plurality of cultivation vessel, particularly in all cultivation vessels, by moving the microscope between a plurality of positions; wherein steps (i) and (ii) may be performed in any order.

In still further embodiments, performing a measurement cycle comprising the steps (ia) determining a first physico-chemical parameter in a plurality of cultivation vessels, particularly in all cultivation vessels, by moving the microscope between a plurality of positions; (ib) determining a second physico-chemical parameter in a plurality of cultivation vessels, particularly in all cultivation vessels, by moving the microscope between a plurality of positions and (ii) imaging the morphology of cells in a plurality of cultivation vessels, particularly in all cultivation vessels, by moving the microscope between a plurality of positons; wherein steps (ia), (ib) and (ii) may be performed in any order.

In certain embodiments, the cultivation media are at least partially exchanged once or several times within a measurement cycle. Time intervals for a partial or complete exchange of cultivation media are usually between about 5 min to about 60 min, particularly between about 10 to about 13 min, e.g. about 20 min. Between consecutive exchanges of cultivation media, a complete measurement cycle may be performed. Alternatively, cultivation media may be exchanged once or several times within a measurement cycle.

According to the present invention, the cells may be cultivated and analyzed without external intervention for a substantial time period, e.g. for a time period of at least about 2 h, at least about 4 h, or at least about 12 h or even longer. For example, the cells may be cultivated and analyzed about 1 d to about 5 d without external intervention.

In certain embodiments, the method is carried out in real-time. In certain embodiments, the method is carried out label free, i.e. without adding labelled compounds to the cultured cells.

The device and the method of the present invention may be used for a plurality of different applications, e.g. for toxicology research, metabolic research, oncology research, drug screening, basic cellular research and/or hypoxia research. In toxicology research, multiple cellular reactions to toxic substances in defined treatment/washout protocols may be monitored. In metabolic research, cellular metabolic profiles in certain protocols, e.g. the metabolic stress test or the glycolytic stress test, may be monitored. In oncology research, reactions of cancer cell lines and primary cancer cells to potential anti-cancer drugs may be monitored. In drug screening applications, a parallel screening of potential drug candidates for the influence on different cell types may be carried out. In hypoxia research, cellular reactions under controlled hypoxic atmospheric conditions may be monitored.

### Particular embodiments

In the following, the present invention is described in more detail by the following embodiments 1-32:
1. Device for the analysis of cultured cells comprising:
   a) a plurality of cultivation vessels adapted for culturing cells, particularly for culturing adherent cells,
   b) a climatic chamber in which the cultivation vessels (a) are positioned,
   c) a supply and/or removal system for supplying and/or removing materials, e.g. fluids, including cultivation media and/or test compounds to or from the cultivation vessels (a), and optionally at least one reservoir for supply materials and for waste,
   d) a light source for transmitting radiation to the cultivation vessels (a),
   e) at least one optical sensor which is responsive to a physico-chemical parameter in a cultivation vessel (a), wherein the physico-chemical parameter is particularly selected from physico-chemical parameters indicative for the metabolic condition of cells, such as pH, oxygen content, and optionally at least one further parameter,
   f) at least one electrode for measuring the impedance of cells in a cultivation vessel (a),
   g) a detector or detector system for receiving radiation from a cultivation vessel (a), wherein the detector or detector system is adapted for (i) determining a physico-chemical parameter in a cultivation vessel (a) and (ii) imaging the morphology of cells in a cultivation vessel (a), and
   h) a microscope for providing an optical path between a cultivation vessel (a) and the detector or detector system (g), wherein the microscope is adapted to be moved between a plurality of positions within the device, wherein an individual microscope position is attributed to an individual cultivation vessel (a), and wherein the microscope comprises switching means for providing a switch between an optical path for determining a physico-chemical parameter and an optical path for imaging the morphology of cells.
2. The device of embodiment 1, which is adapted for performing a measurement cycle comprising the steps (i) determining a physico-chemical parameter and imaging the morphology of cells in a first cultivation vessel (a) and (ii) determining a physico-chemical parameter and imaging the morphology of cells in a second cultivation vessel (a); wherein step (ii) may be repeated until the measurement cycle is performed for all cultivation vessels.
3. The device of embodiment 1 or 2, wherein the switching means is adapted for substantially simultaneous determining a physico-chemical parameter and imaging of cell morphology.
4. The device of embodiment 3 wherein the switching means is adapted for providing a temporal offset between determining a physico-chemical parameter and imaging of cell morphology, which is about 2 s or less, particularly between about 0.5 s and about 1 s.
5. The device of embodiment 3 or 4 wherein the switching means is adapted for providing a spatial offset between determining a physico-chemical parameter and imaging of cell morphology, which is about 2 mm or less, particularly about 0.1 mm and about 1 mm.
6. The device of any one of embodiments 1-5, which is adapted for performing a measurement cycle comprising the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels; wherein the sequence of steps (i), (ii) and (iii) may be repeated at least once until the measurement cycle is performed.
7. The device of embodiment 6, which is adapted for performing a measurement cycle comprising the steps (ia) determining a first physico-chemical parameter in a plurality of cultivation vessels by moving the microscope between a plurality of positions; (ib) determining a second physico-chemical parameter in a plurality of cultivation vessels by moving the microscope between a plurality of positions and (ii) imaging the morphology of cells in a plurality of cultivation vessels by moving the microscope between a plurality of positions; wherein steps (ia), (ib) and (ii) may be performed in any order.
8. The device of any one of embodiments 1-7 wherein the cultivation vessels (a) are located at a stationary position in the device.
9. The device of any one of embodiments 1-8 wherein the microscope (h) comprises an optical switching means, e.g. a switchable optical window.
10. The device of any one of the preceding embodiments wherein the microscope (h) comprises a first optical element and second optical element, and wherein the switching means is adapted for switching between an optical path comprising the first optical element and an optical path comprising the second optical element.
11. The device of embodiment 10 wherein the switching means is adapted for moving the first optical element and the second optical element into or out from the optical path.
12. The device of any one of embodiments 10 to 11 wherein the first optical element and the second optical element are mounted on a movable sliding element.
13. The device of any one of embodiments 10 to 12 wherein the first optical element comprises a prism mirror.
14. The device of any one of embodiments 10 to 13 wherein the second optical element comprises an optically semi-transparent filter or mirror.
15. The device of any one of the preceding embodiments which comprises at least about 6 and up to about 100 cultivation vessels (a), particularly at least about 10 and up to about 30 cultivation vessels (a), and more particularly 24 cultivation vessels (a).
16.The device of any one of the preceding embodiments wherein the cultivation vessels (a) have a culture volume of about 0.1 ml to about 1.5 ml, particularly a culture volume of about 0.5 ml to about 1 ml.
17. The device of any one of the preceding embodiments wherein the climatic chamber (b) is provided with a control element adapted for maintaining and/or adjusting temperature, gas atmosphere, e.g. O₂ partial pressure, and/or humidity.
18. The device of any one of the preceding embodiments wherein the climatic chamber (b) is provided with a separation element, e.g. a slide element, for physically separating the culture vessels from the supply and/or removal system (c) and/or from the microscope (h).
19. The device of any one of the preceding embodiments wherein the climatic chamber (b) is provided with a ventilation filter element.
20. The device of any one of the preceding embodiments wherein the climatic chamber (b) is provided with a pressure control element.
21. The device of any one of the preceding embodiments wherein the supply and/or removal system (c) is adapted for supplying and/or disposing of culture media and/or test substances.
22. The device of any one of the preceding embodiments wherein the supply and/or removal system (c) comprises an automated pipetting system.
23. Use of a device of any one of embodiments 1-22 for cultivating and analyzing cells, particularly for cultivating and analyzing adherent cells, more particularly mammalian cells, e.g. human cells.
24. The use of embodiment 23 for toxicology research, metabolic research, oncology research, drug screening, basic cellular research and/or hypoxia research.
25. A method of cultivating and analyzing cells, comprising the steps:
   a) cultivating cells, particularly adherent cells, in a plurality of cultivation vessels, wherein said cultivation vessel are positioned in a climatic chamber,
   b) supplying and/or removing materials, e.g. fluids, including cultivation media and/or test compounds to or from the cultivation vessels,
   c) transmitting radiation from a light source to the cultivation vessels,
   d) optically determining at least one physico-chemical parameter in the cultivation vessels using an optical sensor which is responsive to said physico-chemical parameter, wherein the physico-chemical parameter is particularly selected from physico-chemical parameters indicative for the metabolic condition of cells, such as pH, oxygen content, and optionally at least one further parameter,
   e) optically imaging the morphology of cells in the cultivation vessels, and
   f) measuring the impedance of cells in the cultivation vessels,
   wherein radiation from a cultivation vessel (a) is transmitted to a detector or detector system, which is adapted for (i) determining a physico-chemical parameter in a cultivation vessel (a) and (ii) imaging the morphology of cells in a cultivation vessel (a),
   wherein an optical path is provided between a cultivation vessel and the detector or detector system,
   wherein the optical path runs via a microscope, which is moved between a plurality of positions within the device, wherein an individual microscope position is attributed to an individual cultivation vessel (a), and
   wherein the microscope comprises switching means for providing a switch between an optical path for determining a physico-chemical parameter and an optical path for imaging the morphology of cells.
26. The method of embodiment 25 wherein a measurement cycle is performed comprising the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels; wherein the sequence of steps (i), (ii) and (iii) may be repeated at least once until the measurement cycle is performed.
27. The method of embodiment 25 or 26 wherein a measurement cycle is performed comprising the steps (i) determining a physico-chemical parameter in a plurality of cultivation vessels by moving the microscope between a plurality of positions and (ii) imaging the morphology of cells in a plurality of cultivation vessels by moving the microscope between a plurality of positions; wherein steps (i) and (ii) may be performed in any order.
28. The method of embodiment 27 wherein a measurement cycle is performed comprising the steps (ia) determining a first physico-chemical parameter in a plurality of cultivation vessels by moving the microscope between a plurality of positions; (ib) determining a second physico-chemical parameter in a plurality of cultivation vessels by moving the microscope between a plurality of positions and (ii) imaging the morphology of cells in a plurality of cultivation vessels by moving the microscope between a plurality of positions; wherein steps (ia), (ib) and (ii) may be performed in any order.
29. The method of any one of embodiments 26-28 wherein cultivation media are at least partially exchanged in time intervals between about 5 min to about 60 min, particularly between about 10 min to about 30 min, e.g. about 20 min.
30. The method of any one of embodiments 25-29 wherein at least one complete measurement cycle is carried out between consecutive exchanges of cultivation media.
31. The method of any one of embodiments 25-30 wherein the cells are cultivated and analyzed for a time period of at least about 2 h, e.g. about 1 d to about 5 d without external intervention.
32. The method of any one of embodiments 25-31 which is carried out in real-time and/or which is carried out label free.

Further, the invention is explained in more detail by the following examples:

### Examples

### Example 1- Mitochondrial Stress Test

The mitochondrial stress test creates a kinetic profile of key parameters of mitochondrial functions by measuring the oxygen consumption rate (OCR) of cells under the influence of different test compounds. Thereby, the basal respiration, ATP production, proton leakage, maximal respiration, reserve capacity, and non-mitochondrial respiration can be defined.

A mitochondrial stress test was performed in a device of the present invention. Mouse fibroblast L929 cells were cultivated in the wells of a micro-titer plate at 37°C and provided with fresh media in time intervals of 20 min.

A measurement cycle involved addition of following compounds at the indicated time points:
t = 60 min: Addition of 1 µM oligomycin A
t = 100 min: Addition of FCCP (carbonyl cyanide p-(trifluoromethoxy) phenylhydrazone) at concentrations of 0 µM (control), 0.13 µM, 0.25 µM or 0.50 µM, respectively.
t = 140 min: Addition of 1 µM antimycin A and 1 µM rotenone

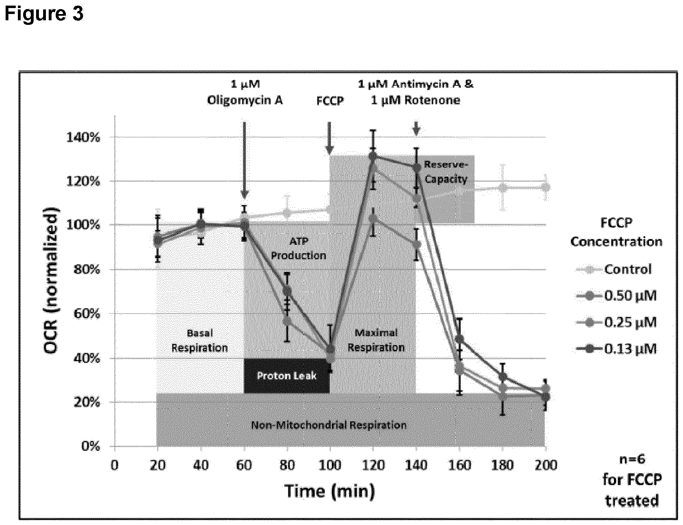

The OCRs for the controls and the treated cells over a time period of 200 min are shown in Fig 3. All OCRs measured were evaluable and consistent. The controls showed a normal course, and all values had only a small standard deviation. Thus, multiple parameters relating to the usable oxygen levels for ATP production can be determined and the maximum respiration of a given cell line can be defined. Further, valuable information on basal respiration, reserve capacity and non-mitochondrial oxygen consumption can be obtained at the same time.

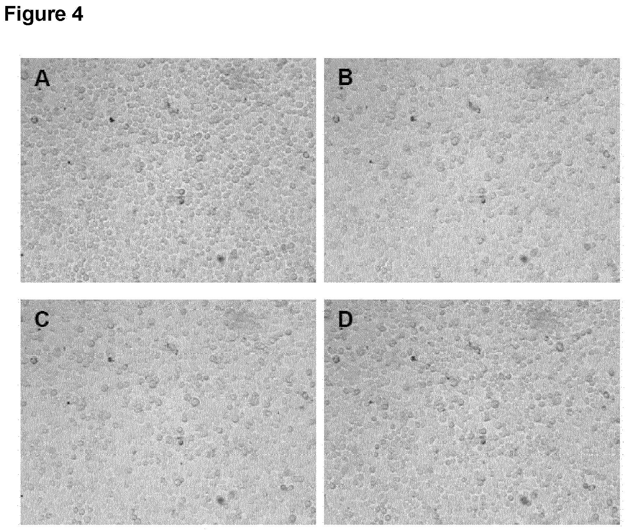

This data were correlated with cell imaging results (Fig. 4). Exemplary images of the morphology of cells belonging to the group with largest changes under FCCP treatment (0.13 µM, well 18) at t = 60 min (Fig. 4A), t = 100 min (Fig. 4B) t = 140 min (Fig. 4C) t = 60 min (Fig. 4D) are shown.

Virtually no significant morphological changes were detected over the measurement period. This corresponds to the expectations since during the measurement cycle of the respiratory chain was influenced only briefly and locally.

The experiment results show an undisturbed, correct course. All measured OCRs were evaluable and consistent. The controls show a normal course.

The different treatment groups differ in their kinetic only at the periods where different FCCP concentrations act on them. In addition, they show only small standard deviations.

The usable oxygen for ATP production is 44% of the basal respiration.

Maximum respiration is within the range of variation under the influence of 0.25 µM FCCP and 0.13 µM FCCP. This shows that the optimal FCCP concentration is in the range of these values. The value is 131% of the basal respiration. Therefore, the cells have a reserve capacity of 31% in this case.

The non-mitochondrial oxygen consumption of the cells is 22%.

Continuous cell imaging reveals vital, sub-confluent cells that show virtually no significant changes over the measurement period.

### Example 2: Toxicology

This assay demonstrates the power of a combined real-time sensor system and a microscope.

L929 mouse fibroblast cells were investigated under the influence of the reference toxicant sodium lauryl sulfate (SLS). The test settings included 12 h of pretreatment and 12 h of treatment with different concentrations of SLS. Throughout the assay, oxygen consumption and extracellular acidification, as well as morphology based on regular microscope images, were recorded.

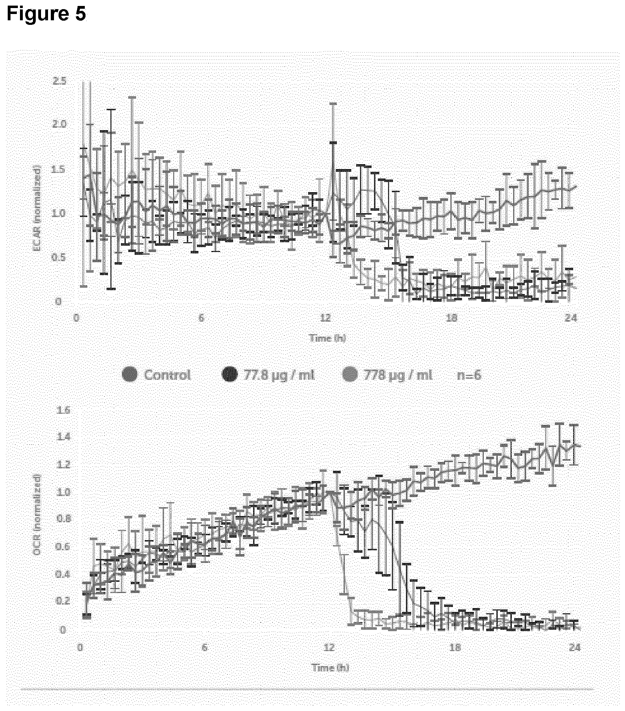

The results of the quantitative metabolic monitoring of the parameters ECAR and OCR over a time period of 24 h are shown in Fig. 5A and B. The medium SLS concentration (77.8 µg/ml) demonstrates how multiple sensing devices can identify the temporal mechanisms of the action of a substance. In the first 3 h after SLS addition, the oxygen uptake was reduced, while acidification increases. Responsible for these opposite effects are rapidly occurring damages to the mitochondrial membranes and the attempt of the cell to compensate for this by increased glycolysis. After continuation of the treatment, the damage to all the cell membranes finally lead to cell death and a drop in all metabolic values.

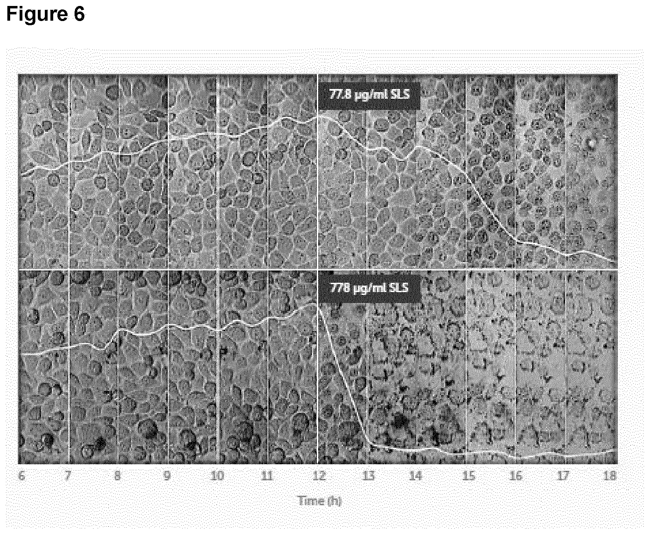

This data can be correlated with cell imaging results (Fig. 6). Because the exact time points of every measuring and imaging event are known, it is possible to correlate these data for comparison. Clear correlations are visible by comparing the OCR with the hourly recorded microscopic images of the cells. OCRs and ECARs of L929 cells under treatment with different concentrations of SLS (77.8 µg/ml, 778 µg/ml) show a clear dose- and time-dependent reaction. After the initial impairment of the respiratory chain, at first the cells try to compensate for this loss by higher glycolytic activity (extracellular acidification).

## Claims

1. Device for the analysis of cultured cells comprising:
a) a plurality of cultivation vessels adapted for culturing cells, particularly for culturing adherent cells,
b) a climatic chamber in which the cultivation vessels (a) are positioned,
c) a supply and/or removal system for supplying and/or removing materials, e.g. fluids, including cultivation media and/or test compounds to or from the cultivation vessels (a), and optionally at least one reservoir for supply materials and for waste,
d) a light source for transmitting radiation to the cultivation vessels (a),
e) at least one optical sensor which is responsive to a physico-chemical parameter in a cultivation vessel (a), wherein the physico-chemical parameter is particularly selected from physico-chemical parameters indicative for the metabolic condition of cells, such as pH, oxygen content, and optionally at least one further parameter,
f) at least one electrode for measuring the impedance of cells in a cultivation vessel (a),
g) a detector or detector system for receiving radiation from a cultivation vessel (a), wherein the detector or detector system is adapted for (i) determining a physico-chemical parameter in a cultivation vessel (a) and (ii) imaging the morphology of cells in a cultivation vessel (a), and
h) a microscope for providing an optical path between a cultivation vessel (a) and the detector or detector system (g), wherein the microscope is adapted to be moved between a plurality of positions within the device, wherein an individual microscope position is attributed to an individual cultivation vessel (a), and wherein the microscope comprises switching means for providing a switch between an optical path for determining a physico-chemical parameter and an optical path for imaging the morphology of cells.

2. The device of claim 1, which is adapted for performing a measurement cycle comprising the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels (a); wherein the sequence of steps (i), (ii) and (iii) may be repeated at least once until the measurement cycle is performed.

3. The device of claim 1 or 2, wherein the switching means is adapted for substantially simultaneous determining a physico-chemical parameter and imaging of cell morphology.

4. The device of claim 3 wherein the switching means is adapted for providing a temporal offset between determining a physico-chemical parameter and imaging of cell morphology, which is about 2 s or less, particularly between about 0.5 s and about 1 s, and/ for providing a spatial offset between determining a physico-chemical parameter and imaging of cell morphology, which is about 2 mm or less, particularly about 0.1 mm and about 1 mm.

5. The device of any one of claims 1-4, which is adapted for performing a measurement cycle comprising the steps (i) determining a physico-chemical parameter in a plurality of cultivation vessels by moving the microscope between a plurality of positions and (ii) imaging the morphology of cells in a plurality of cultivation vessels by moving the microscope between a plurality of positions; wherein steps (i) and (ii) may be performed in any order.

6. The device of claim 5, which is adapted for performing a measurement cycle comprising the steps (ia) determining a first physico-chemical parameter in a plurality of cultivation vessels by moving the microscope between a plurality of positions; (ib) determining a second physico-chemical parameter in a plurality of cultivation vessels by moving the microscope between a plurality of positions and (ii) imaging the morphology of cells in a plurality of cultivation vessels by moving the microscope between a plurality of positions; wherein steps (ia), (ib) and (ii) may be performed in any order.

7. The device of any one of claims 1-6 wherein the cultivation vessels (a) are located at a stationary position in the device.

8. The device of any one of the preceding claims wherein the microscope (h) comprises a first optical element, e.g. a prism mirror, and a second optical element, e.g. an optically semi-transparent filter or mirror, and wherein the switching means is adapted for switching between an optical path comprising the first optical element and an optical path comprising the second optical element,
particularly wherein the switching means is adapted for moving the first optical element and the second optical element into or out from the optical path and wherein the first optical element and the second optical element are mounted on a movable sliding element.

9. The device of any one of the preceding claims wherein the climatic chamber (b) is provided with a control element adapted for maintaining and/or adjusting temperature, gas atmosphere, e.g. O₂ content, and/or humidity, particularly wherein the climatic chamber (b) is further provided with (i) a separation element, e.g. a slide element, for physically separating the culture vessels from the supply and/or removal system (c) and/or from the microscope (h), and/or (ii) a ventilation filter element, and/or (iii) with a pressure control element.

10. Use of a device of any one of claims 1-9 for cultivating and analyzing cells, particularly for cultivating and analyzing adherent cells, more particularly mammalian cells, e.g. human cells.

11. The use of claim 10 for toxicology research, metabolic research, oncology research, drug screening, basic cellular research and/or hypoxia research.

12. A method of cultivating and analyzing cells, comprising the steps:
a) cultivating cells, particularly adherent cells, in a plurality of cultivation vessels, wherein said cultivation vessel are positioned in a climatic chamber,
b) supplying and/or removing materials, e.g. fluids, including cultivation media and/or test compounds to or from the cultivation vessels,
c) transmitting radiation from a light source to the cultivation vessels,
d) optically determining at least one physico-chemical parameter in the cultivation vessels using an optical sensor which is responsive to said physico-chemical parameter, wherein the physico-chemical parameter is particularly selected from physico-chemical parameters indicative for the metabolic condition of cells, such as pH, oxygen content, and optionally at least one further parameter,
e) optically imaging the morphology of cells in the cultivation vessels, and
f) measuring the impedance of cells in the cultivation vessels,
wherein radiation from a cultivation vessel (a) is transmitted to a detector or detector system, which is adapted for (i) determining a physico-chemical parameter in a cultivation vessel (a) and (ii) imaging the morphology of cells in a cultivation vessel (a), wherein an optical path is provided between a cultivation vessel and the detector or detector system,
wherein the optical path runs via a microscope, which is moved between a plurality of positions within the device, wherein an individual microscope position is attributed to an individual cultivation vessel (a), and
wherein the microscope comprises switching means for providing a switch between an optical path for determining a physico-chemical parameter and an optical path for imaging the morphology of cells.

13. The method of claim 12 wherein a measurement cycle is performed comprising the steps (i) determining one or more physico-chemical parameters and imaging the morphology of cells in a first cultivation vessel (a); (ii) determining one or more physico-chemical parameters and imaging the morphology of cells in a second cultivation vessel (a); and (iii) repeating step (ii) for one or more other cultivation vessels (a); wherein the sequence of steps (i), (ii) and (iii) may be repeated at least once until the measurement cycle is performed, or
wherein a measurement cycle is performed comprising the steps (i) determining a physico-chemical parameter in a plurality of cultivation vessels by moving the microscope between a plurality of positions and (ii) imaging the morphology of cells in a plurality of cultivation vessels by moving the microscope between a plurality of positions; wherein steps (i) and (ii) may be performed in any order, or
wherein a measurement cycle is performed comprising the steps (ia) determining a first physico-chemical parameter in a plurality of cultivation vessels by moving the microscope between a plurality of positions; (ib) determining a second physico-chemical parameter in a plurality of cultivation vessels by moving the microscope between a plurality of positions and (ii) imaging the morphology of cells in a plurality of cultivation vessels by moving the microscope between a plurality of positions; wherein steps (ia), (ib) and (ii) may be performed in any order.

14. The method of any one of claims 12-13 wherein cultivation media are at least partially exchanged in time intervals between about 5 min to about 60 min, particularly between about 10 to about 30 min, e.g. at about 20 min,
wherein at least one complete measurement cycle is carried out between consecutive exchanges of cultivation media, and/or
wherein the cells are cultivated and analyzed for a time period of at least about 2 h, e.g. about 1 d to about 5 d without external intervention.

15. The method of any one of claims 12-14 which is carried out in real-time and/or which is carried out label free.
